# EUROPEAN PATENT APPLICATION

(11) **EP 2 623 148 A1**
(43) Date of publication of application: **07.08.2013**
(21) Application number: 11828723.4
(22) Date of filing: 06.09.2011
(51) Int. Cl.: A61M 5/34, A61M 5/28, A61M 5/32

(54) **MEDICAL INSTRUMENT WITH ATTACHED NEEDLE**

(30) Priority: 27.09.2010 JP 2010215363
(71) Applicant: Terumo Kabushiki Kaisha, Tokyo 151-0072 (JP)
(72) Inventor: TACHIKAWA Kouichi, Fujinomiya-shi Shuzuoka (JP); OGAWA Junichi, Nakakoma-gun Yamanashi 409-3853 (JP)
(74) Representative: TBK
(86) International application number: PCT/JP2011/070211
(87) International publication number: WO 2012/043162

(57) **Abstract**

A medical instrument with attached needle of the present invention includes: a needle tube (6) having a needle tip capable of piercing a living body; and a seal member (5) composed of an elastic member and provided to hold an outer surface of the needle tube (6) in a liquid-tight manner in a condition where the needle tip (6a) capable of piercing the living body protrudes from one end and a proximal portion (6b) on a side opposite to the needle tip (6a) protrudes from the other end. The instrument also has: a liquid containing section (3) having a liquid chamber (10) capable of containing a liquid therein; and a needle seating section (2) formed at one end of the liquid containing section (3). The seal member (5) is in contact with an inner surface of the needle seating section (2) and is disposed within the needle seating section (2) in a compressed state. The proximal portion (6b) of the needle tube (6) communicates with the liquid chamber (10).

## Description

### Technical Field

The present invention relates to a medical instrument with attached needle in which a needle is preliminarily attached to one end of a liquid containing section.

### Background Art

In recent years, in view of a problem that glass-made syringes would suffer cracking in a distribution process thereof, plastic-made syringes have come to be used more frequently all over the world.

Conventionally, fixing of a needle to a plastic-made syringe has been carried out mainly by use of an adhesive. In a prefilled syringe in which a syringe is preliminarily filled with a drug, however, such an adhesive may come into contact (liquid contact) with the drug in the syringe, possibly producing a bad influence on the drug. In view of this, in prefilled syringes such as the one described in Patent Document 1, a configuration is generally adopted in which a needle is attached, by high frequency welding, to a syringe or to a needle holding member (needle hub) attached to the tip of the syringe.

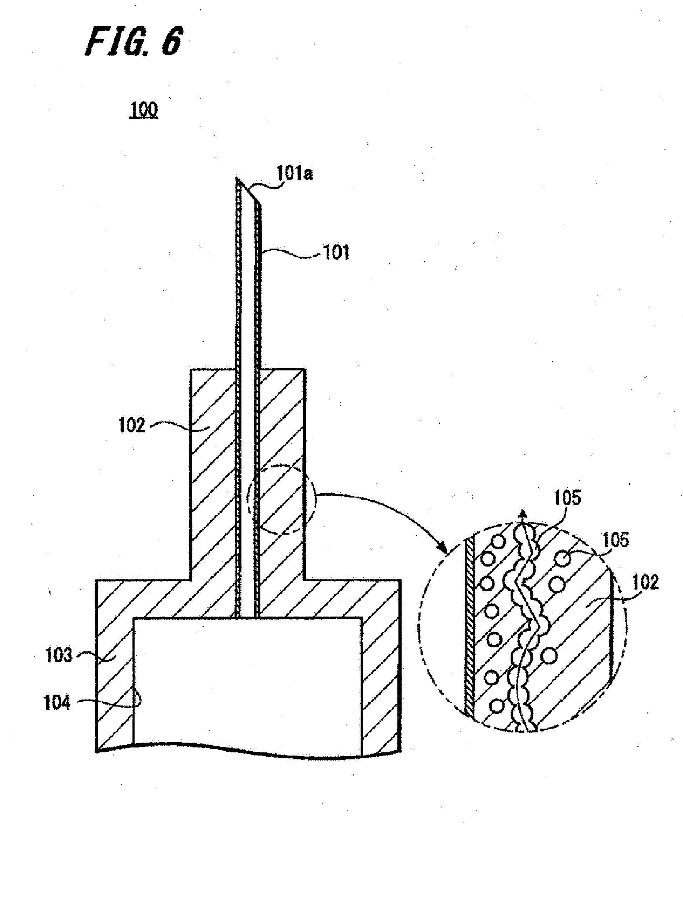

FIG. 6 shows, as an example of the related art, a schematic sectional configuration view and an enlarged view of a major part thereof, illustrating a case where a needle is attached to a tip of a syringe by high frequency welding. As shown in FIG. 6, in the conventional syringe 100, a tubular liquid containing member 103 having a liquid chamber 104 therein is provided at a tip portion (distal end portion) thereof with a cylindrical needle holding section 102 formed to have a diameter smaller than the outside diameter of the liquid containing member 103. In the needle holding section 102, a needle 101 is fixed by the high frequency welding so that a needle tip 101a formed at one end of the needle 101 protrudes from an end face of the needle holding member 102 and that the other end is communicating with the liquid chamber 104.

Meanwhile, in the case of the high frequency welding, a plurality of bubbles 105 are generated in the needle holding section 102, as shown in the enlarged view in FIG. 6. In the case where the bubbles 105 are individually independent bubbles, they do not matter. In the case where the plurality of such bubbles 105 are formed continuously, however, a part where the continuous bubbles 105 are present becomes brittle, and a crack may be generated in the direction of an arrow, as shown in FIG. 6. When the crack is thus generated in the needle holding section 102, the drug contained in the liquid containing member 103 may leak to the exterior. In the high frequency welding, it is difficult to perform such a control that the bubbles 105 generated will be independent, and it is difficult to prevent formation of the continuous bubbles.

### Prior Art Document

### Patent Document

Patent Document 1: Japanese Patent Laid-open No. Hei 08-117334

### Summary of Invention

### Technical Problem

Accordingly, as a method for fixing a needle onto a plastic-made syringe, a new fixing method is needed wherein no bad influence is produced on a drug contained in a syringe and wherein liquid leakage would not occur. Besides, problems of cracking and liquid leakage upon high frequency welding of a needle may occur generally in all of medical instruments with attached needles in which the needle is fixed to a plastic-made needle holding section or member, such as a medical instrument with attached needle which is put to use in the state of being attached to a tip of a tube permitting blood or a medical agent to flow therethrough.

In consideration of the above-mentioned points, it is an object of the present invention to provide a medical instrument with attached needle in which generation of leakage of liquid to the exterior is restrained and enhanced reliability is thereby promised.

### Technical Solution

In order to solve the above-mentioned problems and attain the object of the present invention, there is provided according to the present invention a medical instrument with attached needle which includes a needle tube, a seal member, a needle seating section, and a liquid containing section. The needle tube has a needle tip capable of piercing (puncturing) a living body. The seal member is composed of an elastic member, and provided to hold an outer surface of the needle tube in a liquid-tight manner in a condition where the needle tip protrudes from one end and a proximal portion (base end portion) on the side opposite to the needle tip protrudes from the other end. The liquid containing section is provided on the proximal portion side of the needle tube, and has a liquid chamber communicating with the needle tube. The needle seating section is formed on one end side of the liquid containing section. Besides, the seal member is in contact with an inner surface of the needle seating section and is disposed within the needle seating section in a compressed state, and the proximal portion of the needle tube communicates with the liquid chamber.

In the medical instrument with attached needle according to the present invention, the seal member holding the needle tube is compressed by the needle seating section, whereby a frictional force is generated between the needle tube and the seal member holding the needle tube in a liquid-tight manner. By this frictional force, the needle tube is fixed and held onto the seal member. In addition, since the seal member is held by the needle seating section in a compressed state, an elastic force is generated in the seal member. As a result, liquid-tightness between the seal member and the needle seating section and liquid-tightness between the seal member and the needle tube are enhanced.

### Advantageous Effect

According to the present invention, the needle tube is fixed by a frictional force between the needle tube and the seal member, and the seal member is compressed by the needle seating section. This ensures that liquid-tightness between the seal member and the needle seating section and liquid-tightness between the seal member and the needle tube are maintained. Consequently, the needle tube is maintained, without leakage of liquid to the exterior.

### Brief Description of Drawings

[FIG. 1]

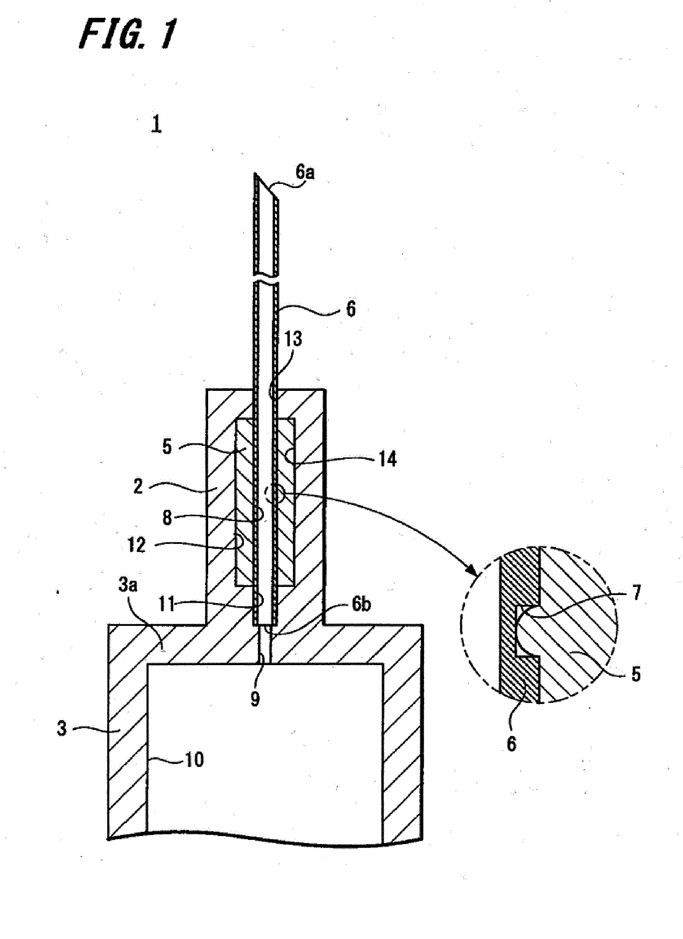

   FIG. 1 is a sectional view showing a configuration of a medical instrument with attached needle according to a first embodiment of the present invention.
[FIG. 2]

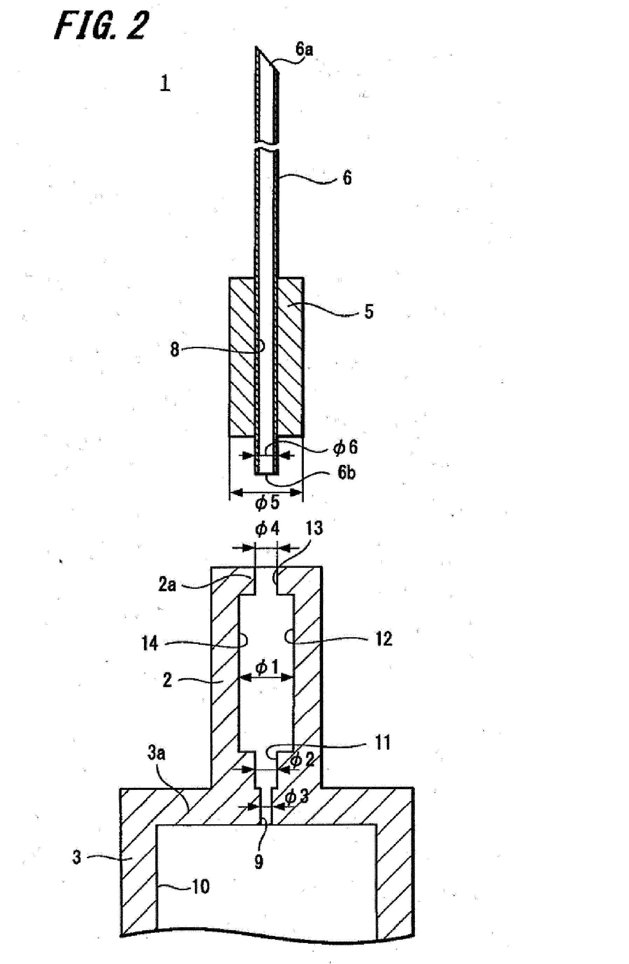

   FIG. 2 is a sectional view showing a pre-assembly state of the medical instrument with attached needle according to the first embodiment of the present invention.
[FIG. 3]

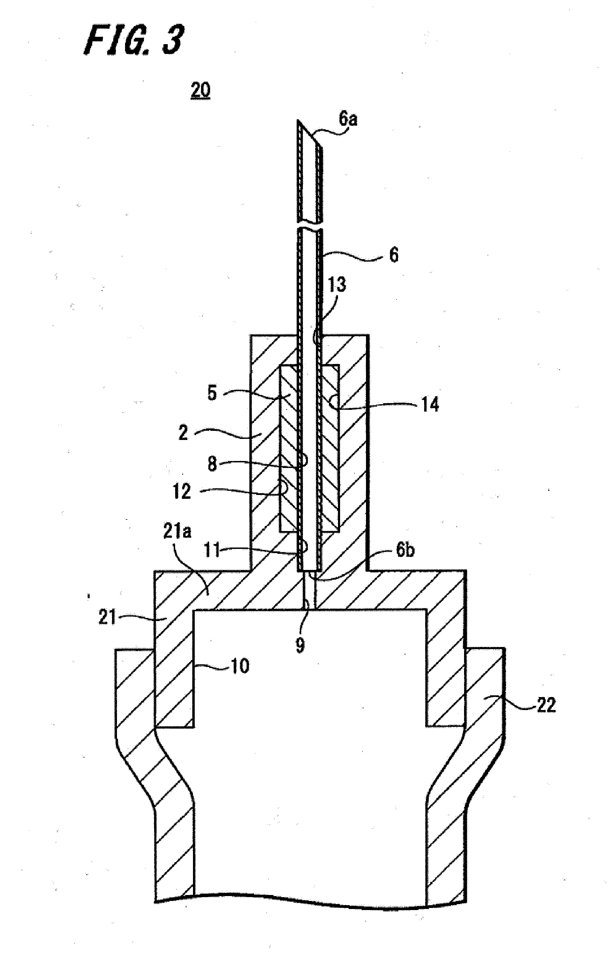

   FIG. 3 is a sectional view showing a configuration of a medical instrument with attached needle according to a modification of the first embodiment of the present invention.
[FIG. 4]

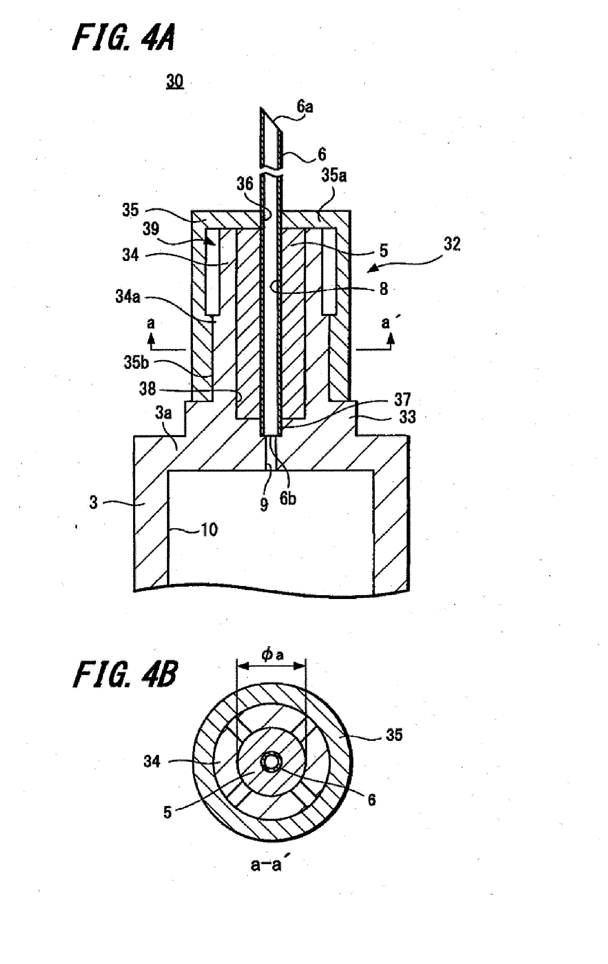

   FIG. 4 shows sectional views illustrating a configuration of a medical instrument with attached needle according to a second embodiment of the present invention.
[FIG. 5]

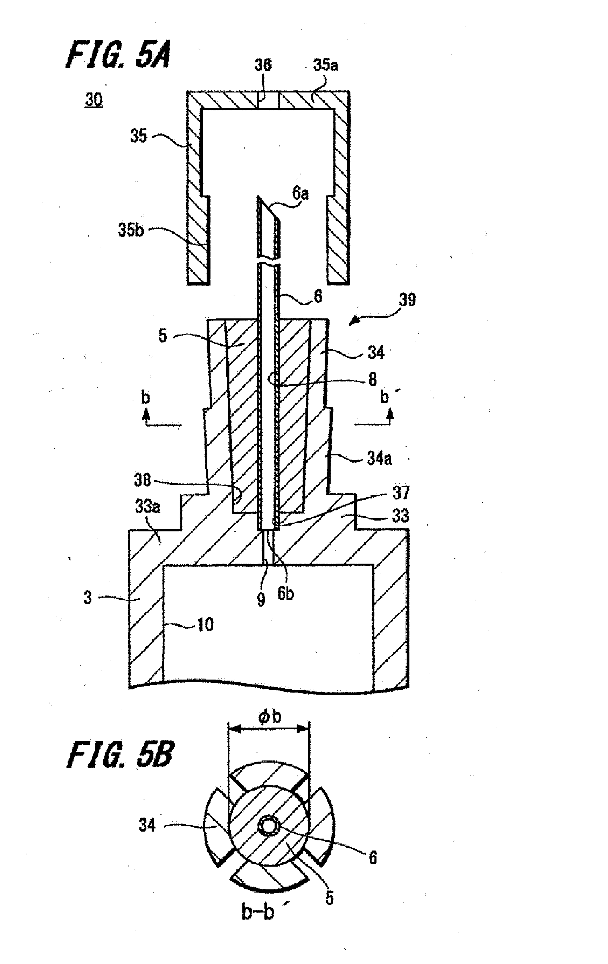

   FIG. 5 shows sectional views illustrating a pre-assembly state of the medical instrument with attached needle according to the second embodiment of the present invention.
[FIG. 6]
   FIG. 6 shows a sectional view and an enlarged view of a major part thereof, illustrating a configuration of a medical instrument with attached needle in an example of the related art.

### Mode for Carrying Out the Invention

Now, some embodiments (exemplary modes) of a medical instrument with attached needle according to the present invention will be described below, referring to FIGS. 1 to 5. Incidentally, members shown in common in the drawings are denoted by the same reference symbols. Besides, the present invention is not limited to the following embodiments (modes).
Incidentally, description will be made in the following order.

### 1. First Embodiment: Medical Instrument with Attached Needle

1-1. Configuration Example of Medical Instrument with Attached Needle
1-2. Method for Assembly of Medical Instrument with Attached Needle
1-3. Modification

### 2. Second Embodiment: Medical Instrument with Attached Needle

2-1. Configuration Example of Medical Instrument with Attached Needle
2-2. Method for Assembly of Medical Instrument with Attached Needle

### <1. First Embodiment: Medical Instrument with Attached Needle>

FIG. 1 is a sectional view showing a configuration of a medical instrument with attached needle 1 according to a first embodiment of the present invention, and FIG. 2 is a sectional view showing a pre-assembly state of the medical instrument with attached needle 1. In this embodiment, a prefilled syringe which is preliminarily filled with a liquid medicine will be described as an example of the medical instrument with attached needle 1.

### [1-1. Configuration Example of Medical Instrument with Attached Needle]

As shown in FIG. 1, the medical instrument with attached needle 1 according to this embodiment includes a needle tube 6, a needle seating section 2 for holding the needle tube 6, a seal member 5, and a liquid containing section 3 having a liquid chamber 10 capable of containing a drug therein.

As the needle tube 6, a needle tube with a size (outside diameter: 3.4 to 0.1 mm) of 10 to 36 gauge, preferably a size (outside diameter: 1.6 to 0.2 mm) of 14 to 33 gauge, according to the ISO medical needle tube standard (IS09626: 1991/Amd. 1:2001(E)) is used. The needle tube 6 is provided at one end thereof with a needle tip 6a having a cutting edge surface. The axial-directionally other end, on the side opposite to the needle tip 6a, of the needle tube 6 will be hereinafter referred to as a proximal portion 6b.

A material of the needle tube 6 may be, for example, stainless steel; however, this is not restrictive, and other metals such as aluminum, aluminum alloys, titanium, and titanium alloys can also be used as the material. In addition, the needle tube 6 is not limited to a straight needle, and may be a tapered needle which is tapered at least at a part thereof. It suffices for the tapered needle to have a proximal portion greater in diameter than a needle tip portion and to have a middle portion of a tapered structure. Besides, the sectional shape of the needle tube 6 is not restricted to a circle but may be a polygon such as a triangle.

In addition, as shown in an enlarged view in FIG. 1, the needle tube 6 is formed with a fine cut (hereinafter referred to as a notched part 7) in a surface thereof. This notched part 7 is formed for enhancing a frictional force between the seal member 5 and the needle tube 6 in an assembled state. The details of it will be described later.

The seal member 5 is composed of a elastic member formed in a cylindrical shape, and is formed along its axis with an insertion hole 8 in which the needle tube 6 is to be inserted and passed. The insertion hole 8 is formed to be smaller than the outside diameter of the needle tube 6. In addition, a surface of the seal member 5 is coated with a silicone oil, for facilitating assemblage.

In the assembled state, the needle tube 6 is inserted and passed in the insertion hole 8 of the seal member 5, with a central portion of the needle tube 6 being enveloped and held in the insertion hole 8, in a condition wherein the needle tip 6a protrudes from one end and wherein the proximal portion 6b on the side opposite to the needle tip 6a protrudes from the other end. The insertion hole 8 is formed to have a diameter smaller than the outside diameter of the needle tube 6. In the assembled state, therefore, when the proximal portion 6b of the needle tube 6 is inserted and passed in the insertion hole 8, the seal member 5 is held in a liquid-tight manner onto the outer surface of the needle tube 6 by an elastic force of its own.

Furthermore, as above-mentioned, the needle tube 6 is formed with the notched part 7 in the surface thereof. As shown in the enlarged view in FIG. 1, therefore, the seal member 5 is fixed by entering into the notched part 7. This enhances a frictional force between the needle tube 6 and the seal member 5, so that the needle tube 6 is fixed by the seal member 5 more firmly. Besides, in this instance, the notched part 7 is covered by the seal member 5, so that the liquid-tightness is maintained between the needle tube 6 and the seal member 5.

As for a material of the seal member 5, the seal member 5 is formed of an elastic material for securing good liquid-tightness between the seal member 5 and the needle tube 6 and good liquid-tightness between the seal member 5 and the needle seating section 2. Examples of the elastic material include various rubber materials such as natural rubber, butyl rubber, isoprene rubber, butadiene rubber, styrene-butadiene rubber, silicone rubbers, and isobutylene rubber, various thermoplastic elastomers based on polyurethane, polyester, polyamide, olefin or styrene, and mixtures of them.

The liquid containing section 3 constitutes a syringe (outer tube) which is filled with a drug, in a prefilled syringe. The liquid containing section 3 is formed in a bottomed tubular shape which is provided with a bottom portion 3a at one end thereof and opened at the other end thereof. A space surrounded by the bottom portion 3a and a gasket (not shown) provided on the opened end side is made to be a liquid chamber 10 which is filled with the drug. The bottom portion 3a of the liquid containing section 3 is formed in its center with a liquid passing hole 9 communicating with a tube hole 14 in the needle seating section 2 which will be described later. The diameter of the liquid passing hole 9 is set to be smaller than the outside diameter of the proximal portion 6b of the needle tube 6.

The needle seating section 2 is formed in a tubular shape, which has an outside diameter smaller than the outside diameter of the liquid containing section 3 and which projects perpendicularly from a surface, on the side opposite to the side for facing the liquid chamber 10, of the bottom portion 3a of the liquid containing section 3. The needle seating section 2 is formed integrally with the liquid containing section 3. Examples of a material(s) for the needle seating section 2 and the liquid containing section 3 include various resins such as polyvinyl chloride, polyethylene, polypropylene, cyclic polyolefins, polystyrene, poly(4-methylpentene-1), polycarbonates, acrylic resins, an acrylonitrile-butadiene-styrene copolymer, polyesters such as polyethylene terephthalate, etc., a butadiene-styrene copolymer, and polyamides (e.g., nylon 6, nylon 6·6, nylon 6-10, nylon 12). Among these, preferred are such resins as polypropylene, cyclic polyolefins, polyesters, and poly(4-methylpentene-1), from the viewpoint of easy molding. Incidentally, it is preferable that the material of the liquid containing section 3 is substantially transparent, for securing inside visibility.

The needle seating section 2 has a tube hole 14 for holding the needle tube 6 held by the seal member 5. In the assembled state, the seal member 5 is placed in the needle seating section 2 in a condition where the seal member 5 is in contact with the inner surface of the tube hole 14. Besides, the seal member 5 is held in a compressed state in the tube hole 14 of the needle seating section 2, and holds the needle hole 6 in a condition where the needle tip 6a capable of piercing a living body protrudes from one end thereof. The tube hole 14 constitutes an insertion port 13, a seal member fixing hole 12, and a needle insertion hole 11 in this order from one end side toward the other end side of the needle seating section 2. The tube hole 14 communicates with the liquid passing hole 9 formed in the bottom portion 3a of the liquid containing section 3.

The insertion port 13 is a part in which the needle tube 6 held by the seal member 5 is inserted at the time of assemblage. The diameter ϕ4 of the insertion port 13 is so set that the seal member 5 can be pressed therein and inserted therethrough. In addition, the diameter ϕ4 of the insertion port 13 is set to be smaller than the diameter ϕ1 of the seal member fixing hole 12, which will be described later. This ensures that, after the seal member 5 is inserted into the seal member fixing hole 12 in the assembled state, the seal member 5 is locked by a projecting part 2a projecting by the difference between the diameter ϕ4 of the insertion port 13 and the diameter ϕ1 of the seal member fixing hole 12; as a result, the seal member 5 is fixed so as not to be dislodged.

The seal member fixing hole 12 is configured in a middle part of the needle seating section 2, and serves as a part for compressing and holding the seal member 5 in the assembled condition. The diameter ϕ1 of the seal member fixing hole 12 is set to be smaller than the outside diameter ϕ5 of the seal member 5 before assembly. The axial length of the seal member fixing hole 12 is set to be approximately equal to the axial length of the seal member 5.

The needle insertion hole 11 is a part into which the proximal portion 6b, protruding from the other end of the seal member 5, of the needle tube 6 is inserted at the time of assemblage. The diameter ϕ2 of the needle insertion hole 11 is set to be slightly larger than the outside diameter of the proximal portion 6b of the needle tube 6. In addition, the depth of the needle insertion hole 11 is set to be approximately equal to the length of protrusion of the proximal portion 6b of the needle tube 6 protruding from the other end of the seal member 5.

The diameter (ϕ1 of the seal member fixing hole 12 is set to be smaller than the outside diameter ϕ5 of the seal member 5 before assembly. This ensures that, when the seal member 5 holding the needle tube 6 is inserted in the tube hole 14, the seal member 5 is contained in the seal member fixing hole 12 in a compressed state. As a result, a frictional force is generated between the seal member 5 and the needle tube 6, whereby the needle tube 6 is fixed to the seal member 5. Besides, the compression of the seal member 5 generates an elastic force in the seal member 5; consequently, the liquid-tightness between the needle seating section 2 and the seal member 5 is enhanced, and the liquid-tightness between the seal member 5 and the needle tube 6 is enhanced.

In addition, in the assembled condition, the proximal portion 6b of the needle tube 6 is inserted in the needle insertion hole 11, whereby the needle tube 6 and the liquid chamber 10 in the liquid containing section 3 are made to communicate with each other through the liquid passing hole 9. The diameter ϕ3 of the liquid passing hole 9 is smaller than the outside diameter of the proximal portion 6b of the needle tube 6; therefore, the needle tube 6 would not protrude into the liquid chamber 10.

Incidentally, in this embodiment, (ϕ1 is 1.2 to 10 mm, preferably 2 to 5 mm; ϕ2 is 0.13 to 3.7 mm, preferably 0.15 to 3.5 mm; ϕ3 is 0.07 to 3.37 mm, preferably 0.09 to 3.2 mm; ϕ4 is 0.8 to 9 mm, preferably 1.0 to 4 mm; and ϕ5 is 1.3 to 12 mm, preferably 2.1 to 8 mm.

### [1-2. Method for Assembly of Medical Instrument with Attached Needle]

Now, a method for assembling the medical instrument with attached needle 1 configured as above will be described below.

First, the needle tube 6 is inserted into and passed through the insertion hole 8 of the seal member 5. A middle portion of the needle tube 6 is held by the seal member 5, in the condition where the needle tip 6a capable of piercing a living body of the needle tube 6 protrudes from one end of the seal member 5 and where the proximal portion 6b of the needle tube 6 protrudes from the other end of the seal member 5.

Next, the needle tube 6 held by the seal member 5 is pressed into the tube hole 14 of the needle seating section 2 from the side of its proximal portion 6b. Since the diameter ϕ4 of the insertion port 13 of the needle seating section 2 is smaller than the outside diameter ϕ5 of the seal member 5 before assembly, the seal member 5 is pressed into the tube hole 14 while in a radially compressed state, at the time of insertion thereof. In this instance, since the surface of the seal member 5 is coated with the silicone oil, insertion of the seal member 5 can be carried out smoothly. Then, the proximal portion 6b of the needle tube 6 is inserted into the needle insertion hole 11 in the needle seating section 2. When the proximal portion 6b reaches an end face of the liquid passing hole 9, the insertion of the needle tube 6 into the needle seating section 2 is completed.

Subsequently, after the seal member 5 holding the needle tube 6 is inserted into the needle seating section 2, as shown in FIG. 1, the liquid chamber 10 of the liquid containing section 3 is filled with a desired drug. Thereafter, a gasket is put in position in a sealed state, whereby the medical instrument with attached needle (prefilled syringe) 1 in this embodiment is completed.

Incidentally, the drug with which the medical instrument with attached needle 1 in this embodiment can be filled may be any drug that is ordinarily used as an injection. Examples of the drug include protein drugs such as antibodies, etc., peptide drugs such as hormones, etc., nucleic acid drugs, cell drugs, blood derivatives, vaccines for prevention of various infectious diseases, carcinostatic agents, anesthetics, narcotics, antibiotics, steroid preparations, proteinase inhibitors, heparin, saccharide injections such as glucose, etc., electrolyte correction injections such as sodium chloride, potassium lactate, etc., vitamin preparations, lipid emulsions, and contrast media.

In this embodiment, the needle tube 6 is held by the seal member 5, and the seal member 5 thus holding the needle tube 6 is held in a compressed state in the needle seating section 2. This ensures that a frictional force is generated between the needle tube 6 and the seal member 5, whereby the needle tube 6 can be fixed on the needle seating section 2. As a result, the needle tube 6 would not slip off from the needle seating section 2. Furthermore, a compressive force is exerted on the seal member 5 from the needle seating section 2, whereby an elastic force is generated in the seal member 5 composed of the elastic member. This enhances the liquid-tightness between the needle seating section 2 and the seal member 5, and the liquid-tightness between the seal member 5 and the needle tube 6. This ensures that the drug contained in the liquid chamber 10 would not leak to the exterior.

In addition, in this embodiment, no adhesive is used for fixing of the needle seating section 2 and the needle tube 6 to each other; therefore, the drug contained in the liquid containing section 3 would not make direct contact with an adhesive. Accordingly, no bad influence would be produced on the drug due to mixing-in of the adhesive. Furthermore, the high frequency welding is also not used for fixing of the needle seating section 2 and the needle tube 6 to each other. Therefore, generation of cracking due to bubbles, as shown in FIG. 6, and the attendant leakage of liquid, can be obviated. Consequently, the medical instrument with attached needle 1 can be used with enhanced reliability.

Besides, in this embodiment, the needle tube 6 is formed with the notched part 7, and the seal member 5 enters into the notched part 7. This ensures that the frictional force between the needle tube 6 and the seal member 5 is further enhanced, so that the needle tube 6
is fixed more firmly. While the example wherein the needle tube 6 is formed with the notched part 7 so as to enhance the frictional force between the needle tube 6
and the seal member 5 has been adopted in this embodiment, another configuration may also be adopted wherein the surface of the needle tube 6 is formed with a minutely rugged part by roughening, thereby enhancing the frictional force between the needle tube 6 and the seal member 5.

In addition, in this embodiment, the diameter ϕ3 of the liquid passing hole 9 is set to be smaller than the outside diameter of the proximal portion 6b of the needle tube 6. This ensures that when a force in the direction from the needle tip 6a side toward the proximal portion 6b of the needle tube 6 is exerted on the needle tube 6, the needle tube 6 can be prevented from retracting toward the liquid chamber 10 side. In the case of using the medical instrument with attached needle 1, a pushing-in force exerted on the needle tube 6 when the needle tube 6 is made to pierce a skin or blood vessel is greater than a pulling-out force generated on the needle tube 6 when the needle tube 6 is drawn out of the skin or blood vessel. With the diameter ϕ3 of the liquid passing hole 9 set to be smaller than the outside diameter of the proximal portion 6b of the needle tube 6, it is ensured that the needle tube 6 can pierce the living body even in the case where the frictional force generated between the needle tube 6 and the seal member 5 at the time of piercing of the skin or blood vessel by the needle tube 6 is very small (or such a frictional force is absent).

Besides, in this embodiment, since the diameter ϕ3 of the liquid passing hole 9 is smaller than the outside diameter of the proximal portion 6b of the needle tube 6, the needle tube 6 is prevented from protruding into the liquid chamber 10. Therefore, when the gasket is moved within the liquid chamber 10 to discharge the drug, the gasket can be moved until it makes contact with the bottom portion 3a of the liquid containing section 3. Consequently, an amount of the liquid left in the liquid chamber 10 can be reduced.

In addition, while the configuration has been adopted in which the seal member 5 is provided along its axis with the insertion hole 8 composed of a hole having a diameter smaller than the outside diameter of the needle tube 6, another configuration may also be adopted in which the insertion hole 8 is composed of a slit and the needle tube 6 is passed through the slit. Thus, the insertion hole 8 formed in the seal member 5 may have any configuration insofar as the needle 6 can be inserted and passed therethrough and the needle tube 6 can be held therein in a secure-contact state. In the case where the slit is formed as the insertion hole 8, the needle tube 6 is inserted after spreading the slit by pressing the seal member 5 from the outer circumference side at the time of inserting and passing the needle tube 6, whereby the needle tube 6 can be easily inserted and passed through the seal member 5.

### [1-3. Modification]

While this embodiment has been described by taking the prefilled syringe as the example of the medical instrument with attached needle 1, a configuration may be adopted in which a needle tube attached to a tip of a tube permitting blood or a medical agent to flow therethrough is attached to the medical instrument with attached needle according to the present invention.
FIG. 3 shows a sectional view of a medical instrument with attached needle 20 according to a modification of the first embodiment. In FIG. 3, the parts corresponding to those in FIG. 1 are denoted by the same reference symbols as used in FIG. 1, and overlapping descriptions of the parts will be omitted.

In the medical instrument with attached needle 20 in the modification, a liquid containing section 21 is configured as a connection section for connection with a tube 22 which is connected to an infusion bag (not shown) or the like.
The liquid containing section 21 is formed in a bottomed tubular shape which has a bottom portion 21a at one end thereof and is opened at the other end thereof. Onto the outer circumference of the opened other end of the liquid containing section 21, the tube 22 composed of an elastic body is fitted in a secure-contact state. Such a configuration is applicable to indwelling needles and blood collection needles.

In the medical instrument with attached needle 20 in the modification, the axial length of the liquid containing section 21 is any length that enables the tube 22 (to be joined) to be stably held on the outer circumferential surface of the liquid containing section 21.

As shown in this modification, also in the medical instrument with attached needle 20 having the needle tube 6 to be attached to the tip of the tube 22, the needle tube 6 is fixed by a frictional force between itself and a seal member 5 composed of the elastic member, and is held on a needle seating section 2 in a liquid-tight manner by an elastic force generated in the seal member 5. Besides, in the medical instrument with attached needle 20 according to the modification, also, fixing of the needle seating section 2 and the needle tube 6 to each other does not need an adhesive or high frequency welding. Therefore, no bad influence is produced on a drug, and leakage of liquid can be prevented from occurring.

### <2. Second Embodiment>

Now, a medical instrument with attached needle 30 according to a second embodiment of the present invention will be described below. FIG. 4A is a sectional view showing a configuration of a medical instrument with attached needle 1 according to a first embodiment of the present invention, and FIG. 4B is a sectional view taken along line a-a' of FIG. 4A. Besides, FIG. 5A is a sectional view showing a pre-assembly state of the medical instrument with attached needle 1, and FIG. 5B is a sectional view taken along line b-b' of FIG. 5A. The medical instrument with attached needle 30 according to this embodiment represents an example (embodiment) which differs from the first embodiment in configuration of a needle seating section. In FIGS. 4 and 5, the parts corresponding to those in FIG. 1 are denoted by the same reference symbols as used in FIG. 1, and overlapping descriptions of the parts will be omitted. In this embodiment, also, a prefilled syringe is described as an example of the medical instrument with attached needle 30.

### [2-1. Configuration of Medical Instrument with Attached Needle]

As shown in FIG. 4A, the medical instrument with attached needle 30 according to this embodiment includes a needle tube 6, a needle seating section 32 for holding the needle tube 6, a seal member 5, and a liquid containing section 3 having a liquid chamber 10 capable of holding a drug therein. The needle seating section 32 includes a support base 33, a clamping part 39, and a cap part 35.

The support base 33 is provided in a central area on a surface, on the side opposite to the liquid chamber 10, of a bottom portion 3a of the liquid containing section 3. The support base 33 is composed of a cylindrical member formed to project perpendicularly to the surface of the bottom portion 3a. An end face, on the side opposite to the liquid containing section 3 side, of the support base 33 is provided with a seal member fixing groove 38 in which the other end of the seal member 5 is inserted and held in a fixed manner. Further, in a central area of the bottom surface of the seal member fixing groove 38, a needle insertion hole 37 is provided which communicates with a liquid passing hole 9 formed in the bottom portion 3a of the liquid containing section 3 and in which a proximal portion 6b of the needle tube 6 is held in a fixed manner.

The diameter of the seal member fixing groove 38 is set to be slightly smaller than the outside diameter of the seal member 5 before assembly. It suffices for the depth of the seal member fixing groove 38 to be at such a level that the other end of the seal member 5 is fixed by being partly fitted into the seal member fixing groove 38. The depth of the seal member fixing groove 38 may be about 3 to 15 mm; for example, the depth may be 8 mm. In addition, the diameter of the needle insertion hole 37 is set to be slightly larger than the diameter of the proximal portion 6b of the needle tube 6 so that the proximal portion 6b of the needle tube 6 can be inserted in the needle insertion hole 37. The depth of the needle insertion hole 37 is set to be equal to the length of protrusion of the proximal portion 6b of the needle tube 6 protruding from the other end of the seal member 5. The diameter of the needle insertion hole 37 may be about ϕ6 + 0.03 to ϕ6 + 0.3 mm, and may be, for example, ϕ6 + 0.05 mm, where ϕ6 is the outside diameter of the needle tube 6.

The clamping part 39 is formed to project perpendicularly from the end face, on the side opposite to the liquid containing section 3 side, of the support base 33. The clamping part 39 is composed of a plurality of (in FIGS. 4 and 5, four) column clamping part main bodies 34 formed to surround the seal member fixing groove 38. The clamping part main bodies 34 each have a shape obtained by segmenting a cylindrical member along the axial direction, and are arranged in the surroundings of the seal member fixing groove 38 at regular intervals along the circular circumference. In an assembled condition, the seal member 5 holding the needle tube 6 is inserted in a region surrounded by the four clamping part main bodies 34, and the seal member 5 is thus clamped between the four clamping part main bodies 34.

The inside diameter of the clamping part 39 composed of the four clamping part main bodies 34, before assembly, is set to be equal to or slightly smaller than the outside diameter of the seal member 5 before assembly. In addition, the height of the clamping part main bodies 34 is so set that the height from the bottom surface of the seal member fixing groove 38 to the tips of the clamping part main bodies 34 is approximately equal to the axial length of the seal member 5. Furthermore, side surfaces, on the side opposite to the side for clamping the seal member 5, of the clamping part main bodies 34 are formed with projected parts 34a projected radially outward from the support base 33 to axial-directionally middle portions of the clamping part main bodies 34.

The cap part 35 is composed of a bottomed tubular member which has a bottom portion 35a at one end thereof and is opened at the other end thereof. The bottom portion 35a is formed in a central area thereof with a through-hole 36 through which the needle tube 6 can be passed. The depth of the cap part 35 from the opened end face to the bottom portion 35a is set to be equal to the height of the clamping part main bodies 34 from the support base 33. The cap part 35 is formed, at an inner circumferential surface thereof, with a reduced diameter part 35b formed to project radially inward, from the opened end to a middle portion. The inside diameter of the cap part 35 at a position where the reduced diameter part 35b is formed, before assembly, is set to be smaller than the outside diameter of the clamping part 39 at a position where the projected part 34a is formed.

In an assembled state, as shown in FIGS. 4A and 4B, the cap part 35 is fitted so that its reduced diameter part 35b makes contact with the projected parts 34a of the clamping part main bodies 34. The inside diameter of the cap part 35 at the reduced diameter part 35b, before assembly, is set to be smaller than the outside diameter of the clamping part 39 at a position where the projected part 34a is formed. When the cap part 35 is fitted onto the clamping part 39, therefore, each of the clamping part main bodies 34 is pressed against the seal member 5. This ensures that a compressive force from the outer circumferential surface is generated in the seal member 5. Accordingly, the outside diameter ϕa of the seal member 5 in the assembled state is smaller than the outside diameter ϕb of the seal member 5 before the fitting of the cap part 35.

In addition, though omitted in the drawing of FIG. 4A, in this embodiment, like in the first embodiment, a notched part is formed at the surface of the needle tube 6, so as to enhance the frictional force between the seal member 5 and the needle tube 6.

### [2-2. Method for Assembly of Medical Instrument with Attached Needle]

Now, a method for assembling the medical instrument with attached needle 1 configured as above will be described below.

First, the needle tube 6 is inserted and passed through the insertion hole 8 of the seal member 5, and a middle portion of the needle tube 6 is held by the seal member 5 in the condition where the needle tip 6a capable of piercing a living body, of the needle tube 6, protrudes from one end of the seal member 5 and the proximal portion 6b of the needle tube 6 protrudes from the other end of the seal member 5.
Then, the needle tube 6 held by the seal member 5 is inserted into the region surrounded by the clamping part main bodies 34, from the tip side of the clamping part 39.

Here, the inside diameter of the clamping part 39 composed of the plurality of clamping part main bodies 34, before assembly, is set to be equal to or smaller than the outside diameter of the seal member 5 before assembly. In this case, since the clamping part main bodies 34 are each formed into a column shape, they can be easily bent radially outward. Therefore, the seal member 5 can be smoothly inserted into the region surrounded by the clamping part main bodies 34. In addition, the surface of the seal member 5 is coated with the silicone oil, so that the insertion of the seal member 5 can be carried out in a smooth manner.

Then, the proximal portion 6b of the needle tube 6 is inserted into the needle insertion hole 379, and the other end of the seal member 5 is urged into the seal member fixing groove 38. Since the diameter of the liquid passing hole 9 is smaller than the outside diameter of the proximal portion 6b of the needle tube 6, the insertion of the needle tube 6 into the clamping part 39 is stopped when the proximal portion 6b of the needle tube 6 reaches the end face of the liquid passing hole 9.

In this instance, since the diameter of the seal member fixing groove 38 is set to be slightly smaller than the diameter of the seal member 5 before assembly, the other end of the seal member 5 is inserted in the seal member fixing groove 38 in a compressed state. Therefore, the seal member 5 is held in the seal member fixing groove 38 in the state of being fixed to a certain extent by the elastic force of the seal member 5.

Thereafter, the cap part 35 is so moved that the clamping part 39 is inserted therein from the opening side thereof, the needle tip 6a of the needle tube 6 is passed through the through-hole 36 in the bottom portion 35a, and the end face on the opening side of the cap part 35 is abutted on the end face of the support base 33. As a result, the cap part 35 is fitted onto the clamping part 39 so that the reduced diameter part 35b of the cap part 35 makes contact with the projected parts 34a of the clamping part main bodies 34.

Here, the fixing in fitting the cap part 35 and the clamping part 39 onto each other can be performed by a method wherein the cap part 35 is formed with a male screw at the inner circumference thereof, whereas the clamping part 39 is formed with a female screw at its side surface on the side opposite to the side for facing the seal member 5, and the cap part 35 and the clamp part 39 are put into screw engagement with each other. Alternatively, the fixing may be conducted by a method wherein the cap part 35 is formed at the inner circumference thereof with a lock claw or claws capable of locking with a side surface, on the side opposite to the side for facing the seal member 5, of the clamping part 39, and the lock claw or claws are locked with the side surface. Further, the fixing of the cap part 35 and the clamping part 39 may be performed by use of an adhesive. However, the fixing is preferably performed by the screw engagement or the locking, for avoiding mixing of the adhesive into the drug contained in the liquid chamber 10.

Then, after the cap part 35 is fitted and connected to the clamping part 39, the liquid chamber 10 of the liquid containing section 3 is filled with a desired drug, in the same manner as in the first embodiment. Thereafter, the gasket is placed in position in a sealed manner. As a result, the medical instrument with attached needle (prefilled syringe) 30 in this embodiment is completed.

In this embodiment, the clamping part main bodies 34 constituting the clamping part 39 are each formed into a column shape, so that they can be bent more easily as compared with the case where a hollow cylindrical member constitutes the clamping part 39. Therefore, the clamping part main bodies 34 can each be widened radially outward. This ensures that when the seal member 5 holding the needle tube 6 is inserted into the region surrounded by the clamping part main bodies 34, the insertion of the seal member 5 can be carried out in the condition where a compressive force exerted on the seal member 5 is small. Then, when the cap 35 is fitted in position, the clamping part main bodies 34 are bent to the side for making contact with the seal member 5, so that the seal member 5 can be compressed.

In this embodiment, with the cap part 35 fitted onto the clamping part 39, the clamping part 39 is fixed in the state of being pressed against the seal member 5. Therefore, a compressive force is exerted on the seal member 5. As a result, the frictional force between the needle tube 6 and the seal member 5 is increased, and the needle tube 6 is fixed to the seal member 5 by this frictional force. In addition, with the compressive force exerted on the seal member 5, an elastic force is generated in the seal member 5. This ensures that liquid-tightness between the seal member 5 and the needle tube 6 and liquid-tightness between the seal member 5 and the clamping part 39 are enhanced, so that the drug contained in the liquid containing section 3 is prevented from leaking to the exterior.

Besides, in this embodiment, an other-end-side portion of the seal member 5 is inserted in the seal member fixing groove 38 of the support base 33, thereby being fixed. Therefore, the liquid-tightness at a portion, near the liquid fixing section 3, of the seal member 5 is further enhanced, so that the liquid leakage-preventing effect is more enhanced.
In addition, effects which are the same as or similar to those obtained in the first embodiment can be obtained.

While the clamping part main bodies 34 have been formed with the projected parts 34a and the cap part 35 has been formed with the reduced diameter part 35b in this embodiment, various modifications are possible insofar as the clamping part 39 is pressed against the seal member 5 when the cap 35 is fitted onto the clamping part 39. For example, a configuration may be adopted in which side surfaces of the clamping part main bodies 34 or the inner circumferential surface of the cap part 35 is formed in a tapered shape so that the clamping part 39 is pressed against the seal member 5 when the cap part 35 is fitted onto the clamping part 39.

The medical instrument with attached needle 30 according to this embodiment, also, is applicable to medical instruments with attached needle which are to be used by being attached to a tip of a tube permitting a medical agent or blood to flow therethrough, like the one in the modification of the first embodiment. In this case, effects which are the same as or similar to those obtained in the modification of the first embodiment can be obtained.

In the foregoing, some embodiments of the medical instrument with attached needle according to the present invention have been described together with the operations and effects thereof. However, the medical instrument with attached needle according to the present invention is not restricted to the above-described embodiments, and various modifications on a carrying-out basis are possible within the scope of the invention as defined by the claims.

### Industrial Applicability

The present invention is applicable to injectors, indwelling needles, and other medical instruments with attached needles.

### Explanation of Reference Symbols

1, 20, 30 ... Medical instrument with attached needle, 2, 32 ... Needle seating section, 2a ... Projecting part, 3, 21 ... Liquid containing section, 3a ... Bottom portion, 5 ... Seal member, 6 ... Needle tube, 6a ... Needle tip, 6b ... Proximal portion, 7 ... Notched part, 8 ... Insertion hole, 9 ... Liquid passing hole, 10 ... Liquid chamber, 11 ... Needle insertion hole, 12 ... Seal member fixing hole, 13 ... Insertion port, 14 ... Tube hole, 22 ... Tube, 33 ... Support base, 34 ... Clamping part main body, 35 ... Cap part, 35a ... Bottom portion, 35b ... Reduced diameter part, 36 ... Through-hole, 37 ... Needle insertion hole, 38 ... Seal member fixing groove, 39 ... Clamping part

## Claims

1. A medical instrument with attached needle, comprising:
a needle tube having a needle tip capable of piercing a living body;
a liquid containing section having a liquid chamber capable of containing a liquid therein;
a needle seating section formed at one end of the liquid containing section; and
a seal member composed of an elastic member and provided to hold an outer surface of the needle tube in a liquid-tight manner in a condition where the needle tip and a proximal portion on a side opposite to the needle tip are protruding,
wherein the seal member is in contact with an inner surface of the needle seating section and is disposed within the needle seating section in a compressed state, and the proximal portion of the needle tube communicates with the liquid chamber.

2. The medical instrument with attached needle according to claim 1,
wherein the liquid containing section is formed with a liquid passing hole through which the needle tube and the liquid chamber communicate with each other, and a diameter of the liquid passing hole is smaller than an outside diameter of the proximal portion of the needle tube.

3. The medical instrument with attached needle according to claim 1,
wherein a surface of the needle tube is formed with a finely notched part or rugged part for enhancing a frictional force between the needle tube and the seal member.

4. The medical instrument with attached needle according to claim 1,
wherein the needle seating section includes a plurality of clamping parts each formed into a column shape for clamping the seal member, and a cap part fitted to the clamping parts so as to press the clamping parts against the seal member.

5. The medical instrument with attached needle according to claim 1,
wherein the liquid containing section is composed of a syringe prefilled with a drug.
